# EUROPEAN PATENT SPECIFICATION

(11) **EP 4 458 277 B1**
(45) Date of publication and mention of the grant of the patent: **18.02.2026**
(21) Application number: 23382407.7
(22) Date of filing: 02.05.2023
(51) Int. Cl.: A61B 17/00, A61B 90/00, B65D 83/00, B65D 81/32, C08F 2/01

(54) **METHOD FOR ACTIVATING OLEFIN-BASED MONOMERS AND DEVICE FOR DISPENSING AN OLEFIN-BASED TISSUE ADHESIVE**
VERFAHREN ZUR AKTIVIERUNG VON MONOMEREN AUF OLEFINBASIS UND VORRICHTUNG ZUR AUSGABE EINES GEWEBEKLEBSTOFFS AUF OLEFINBASIS
PROCÉDÉ D'ACTIVATION DE MONOMÈRES À BASE D'OLÉFINE ET DISPOSITIF DE DISTRIBUTION D'UN ADHÉSIF TISSULAIRE À BASE D'OLÉFINE

(43) Date of publication of application: 06.11.2024
(73) Proprietor: Synkotech Biocompatible Materials, S.L., 43204 Reus (Tarragona) (ES)
(72) Inventor: Toral Milian, Marina, 43204 Reus (Tarragona) (ES); Balanta Castillo, Angélica, 43204 Reus (Tarragona) (ES); de la Fuente Molina, Verónica, 43204 Reus (Tarragona) (ES); Bonet Laplana, Amadeu, 43204 Reus (Tarragona) (ES)
(74) Representative: Araujo, Daniel

(56) References cited:
- WO-A1-2015/089106
- WO-A2-01/32319
- US-A1- 2005 175 395

## Description

### Technical Field

The present invention relates to a device for dispensing a tissue adhesive and a method for activating a monomeric and/or polymeric composition.

In particular, the invention relates to a device for dispensing a tissue adhesive, such as a medical adhesive, and a method for activating a monomeric and/or polymeric composition comprising at least one electron-deficient olefin-based monomer, by means of an activator solution arranged in a discontinuous geometrically symmetrical pattern on and/or in at least one porous element.

### Background

Tissue adhesives are commonly used as skin protectants to prevent or treat skin irritation caused by moisture, friction, and shear and as surgical glues to close wounds in substitution of staples or surgical sutures and/or in combination with additional dressings.

Medical adhesives may be used in a variety of settings, including hospitals, long-term care facilities, and home health care.

Cyanoacrylates have been dominating the field of medical adhesives due to their ability to form a strong and durable bond with the skin. Cyanoacrylates are very reactive monomers and activation is very straightforward. Highly active cyanoacrylates, such as butyl cyanoacrylate, work by reacting with the moisture on the skin's surface, which causes the adhesive to polymerize and form a hard, durable bond. Other bulkier cyanoacrylates, such as octyl cyanoacrylate, require of the presence of an activator to polymerize. Such an activator is usually placed on the top surface of a UHMWPE filter, in particular a salt (potassium salt or ammonium salt), and the monomer is activated when passing through the filter.

EP1250263B1 and EP0832137B1 disclose applicators for dispensing a synthetic or semisynthetic polymerizable or cross-linkable monomer material, in particular cyanoacrylates, comprising a porous applicator tip or porous material loaded with an accelerator.

Olefins are less reactive than cyanoacrylates and cannot be activated by the same means as cyanoacrylates because they lead to incomplete/partial polymerization which promotes cytotoxicity, adhesion to other materials after curing and low tissue adhesion, among other issues.

Olefin-based tissue adhesives are a type of adhesives designed to protect the skin or hold together an open wound to naturally close with a better cosmetic output than traditional surgical sutures. These products typically contain one or more types of olefin-based monomers, which are compounds that can polymerize (i.e., form a solid film) when exposed to an activator.

Olefins are compounds that contain a carbon-carbon double bond, and can be either electron deficient or electron rich, depending on the specific chemical structure of the molecule. In the context of tissue adhesives, olefin-based monomers that are used to create a solid film are typically electron deficient. These monomers have a chemical structure that includes one or more electron-withdrawing groups, which help to make the double bond more reactive and facilitate polymerization when the monomer is exposed to an activator. Examples of electron-deficient olefin-based monomers commonly used in tissue adhesive formulations include methyl methacrylate and methylene malonate.

EP2831125B1 discloses a polymerizable system and a method of initiating polymerization comprising a methylene malonate and a polymerization activator which is able to initiate polymerization upon contact with the polymerizable composition without substantial mixing. However, although this method allows polymerization of the composition, polymerization is not reproducible and results in the formation of a diverse range of films having large variability in terms of physical and adhesion properties.

WO 01/32319 A2 discloses a device for dispensing a tissue adhesive according to the preamble of claim 1.

There is, therefore, a need for a device and method for dispensing an olefin-based tissue adhesive that can be easily applied, efficiently activated, that provides a complete and consistent polymerization, in a controlled manner, for a long-lasting intended use.

### Summary

The device and method of the present invention utilize an activator solution arranged on and/or in a porous element to activate an electron-deficient olefin-based monomer, having at least one electron-withdrawing group, comprised in a monomeric and/or polymeric composition to solidify on dry or wet tissue and form a solid film.

The porous element allows the monomeric and/or polymeric composition to pass therethrough and activate the electron-deficient olefin-based monomer to initiate polymerization.

The porosity of the porous element can be tailored in such a manner that depending on the viscosity of the monomeric and/or polymeric composition it ensures a minimum residence time of the latter in the porous element, such that it contacts and mixes with the activator solution sufficiently to initiate polymerization.

The activation can be modulated depending on the geometrical disposition of the activator solution on and/or in the porous element, the number of porous elements, the porosity of the porous element, the viscosity of the monomeric and/or polymeric composition, the reactivity of the monomeric and/or polymeric composition, and the additives present in the monomeric and/or polymeric composition and/or in the porous element.

The activator solution is loaded on and/or in the at least one porous element in a predetermined discontinuous geometrically symmetrical pattern. The activator solution may be applied in a multiple number of discontinuous geometrically symmetrical patterns.

### Brief description of the drawings

FIG. 1 is a perspective exploded view of the device according to an embodiment of the present invention.
FIG. 2 is a perspective exploded view of the device according to an alternate embodiment of the present invention.
FIG. 3 is a perspective exploded view of the device according to a further embodiment of the present invention.
FIG. 4 shows different arrangements of an activator loaded on and/or in a porous element.

### Detailed description

The present invention provides a device for dispensing a tissue adhesive, said device comprising an outer body 1 made of a flexible material, a monomeric and/or polymeric composition 3 arranged inside said outer body 1, an applicator tip 4 removably attached to said outer body 1 and at least one porous element 5 comprised inside the applicator tip 4 and having an activator solution 6 arranged thereon and/or therein.

The monomeric and/or polymeric composition 3 comprises at least one electron-deficient olefin-based monomer, having at least one electron-withdrawing group, such as a methylene malonate monomer, and the activator solution 6 comprises at least one activator.

Examples of electron-deficient olefin-based monomers according to the invention include, but are not limited to, monomers having the following general formula: 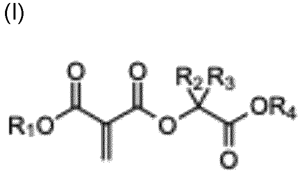 wherein;

| R1 | R2 | R3 | R4 |
|---|---|---|---|
| ethyl | H | H | ethyl |
| ethyl | Me | H | ethyl |
| ethyl | Me | Me | ethyl |
| allyl | H | H | ethyl |
| ethyl | H | H | allyl |
| butyl | H | H | ethyl |
| hexyl | H | H | ethyl |
| ethyl | H | H | MEK |

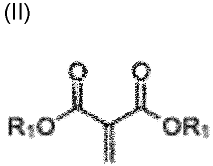

wherein R1: ethyl, ethyl glycol; 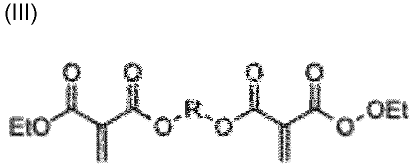 wherein R: butyl; and 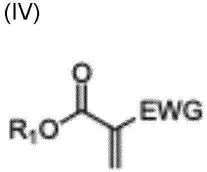 wherein,

| EWG | R1 |
|---|---|
| CN | butyl |
| CN | hexyl |
| CN | octyl |
| Ethyl ketone | ethyl |
| Isopropyl ester | ethyl |

The activator solution 6 comprises at least an activator selected from a compound having a lone pair availability such as, but not limited to, amines, phosphine and ate complex such boronic ate complex.

The activator solution 6 may further comprise an additive or combination of additives.

As used herein, the term "additives" refers to additives used to enhance physical or chemical properties of the tissue adhesive and to provide a desired result. Such additives include, but are not limited to, dyes, plasticizing agents, organic solvents or co-solvents, adhesion promoters and/or stabilizers.

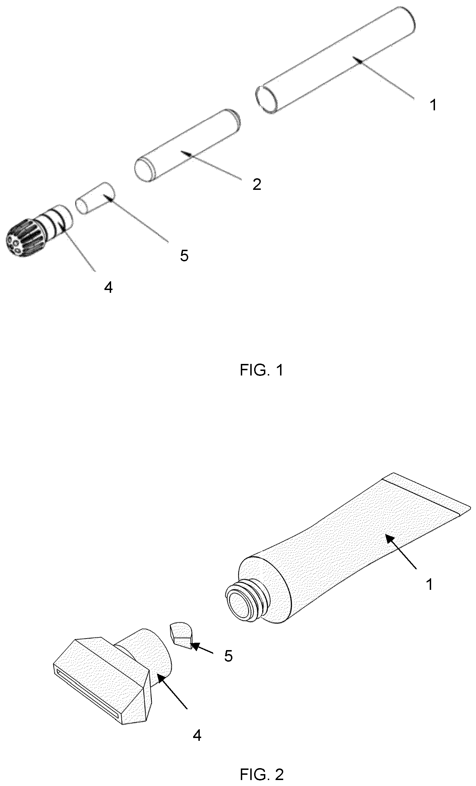

In one embodiment, as shown in Fig. 1, the monomer and/or polymeric composition 3 is comprised inside a frangible container 2 arranged inside said outer body 1 such that, a user, upon applying stress on the outer body 1, is able to break the frangible container 2, release the monomer and/or polymeric composition 3 and force the latter through the at least one porous element 5 and through the applicator tip 4, such that the monomer and/or polymeric composition 3 may react with the activator solution 6 loaded on and/or in the at least one porous element 5 as it passes through the latter.

The frangible container 2 is a hermetically sealed and leak-proof container.

In such an embodiment, preferably, the outer body 1 is made of a flexible plastic material.

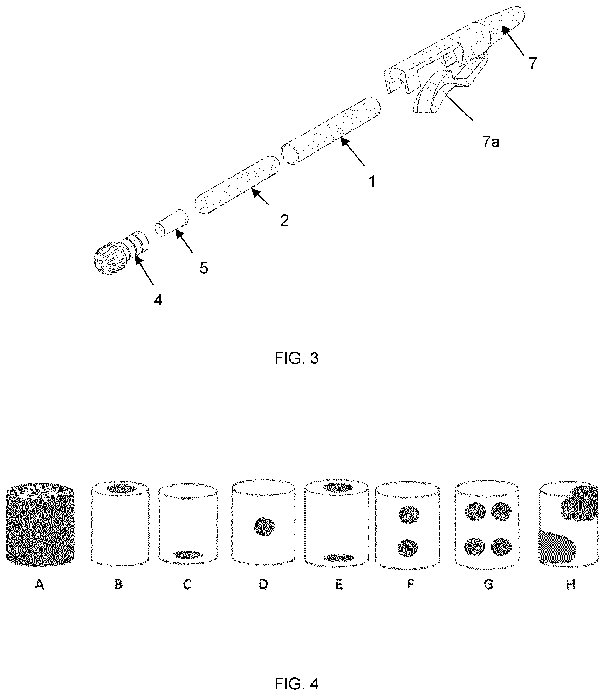

It has also been contemplated that such a device comprises an accessory 7 arranged over said outer body 1 and comprising at least one pressure arm 7a for exerting a controllable pressure over said outer body 1, as shown in Fig. 3, to enable a more precise and accurate dispensation when said device is used in wound closure.

In an alternate embodiment, as shown in Fig. 2, the outer body 1 is made of a flexible metal material, such that, a user, upon applying stress on the outer body 1 is able to force the monomer and/or polymeric composition 3 through the porous element 5 arranged inside the applicator tip 4.

In such an embodiment, preferably, the flexible metal material is aluminium.

According to the invention, the porous element 5 may have a cylindrical shape, or any other shape configured to provide a tight fit between the latter and the applicator tip 4.

In an embodiment of the present invention, the applicator tip 4 has a dovetail configuration and the at least one porous element 5 has a configuration having a minor end and a major end, the major end having a larger width than the minor end, such that the porous element has a taper in width from the major end to the minor end to provide a tight fit between the porous element 5 and the applicator tip 4.

The applicator tip 4 has a distal end comprising at least one opening 7.

The applicator tip 4 is attached to the outer body 1 by pressure, friction fitting, threads or luer locks.

According to the invention, the activator solution 6 is arranged on and/or in the at least one porous element 5 in a predetermined discontinuous geometrically symmetrical pattern that ensures an even and efficient activation of the monomeric and/or polymeric composition 3 throughout each dispensation.

The at least one porous element 5 is made of an inert porous plastic material. Examples of inert porous plastic materials are, but not limited to, UHMWPE, PE, PP, polyurethane and cellulose acetate.

As used herein, the term "discontinuous" refers to providing areas in and/or on the porous element that are not covered by the activator solution, such as by the activator solution being provided in the form of regular spots, lines, or the like, where the activator solution does not cover the entire surface and volume of the porous element.

As used herein, "geometrically symmetrical" refers to the activator solution being arranged symmetrical including, but not limited to, reflectional symmetry, rotational symmetry, translational symmetry, helical symmetry and scale symmetry.

As shown in FIG. 3, type A, B, C and D patterns are analogous to prior art devices used for cyanoacrylate activation, wherein either the porous element is continuously and fully loaded with an activator or the porous element is discontinuously loaded with an activator but without having a predetermined geometrically symmetrical distribution. In particular, type A pattern corresponds to a continuously fully loaded porous element, type B corresponds to a porous element having an activator arranged at a predetermined spot on its top surface, type C corresponds to a porous element having an activator arranged at a predetermined spot on its bottom surface and type D corresponds to a porous element having an activator arranged at a predetermined spot on its lateral surface.

Type E to H patterns correspond to porous elements 5 comprising an activator solution 6 arranged thereon and/or therein following a discontinuous geometrically symmetrical pattern, according to different embodiments of the present invention. In particular, type E corresponds to a porous element 5 having an activator solution 6 arranged at a predetermined area on both, top and bottom surfaces; type F corresponds to a porous element 5 having an activator solution 6 arranged at two geometrically symmetrical spots on its lateral surface with respect to a transverse symmetry plane that divides the porous element approximately into two mirror-identical halves, type G corresponds to a porous element 5 having an activator solution 6 arranged at four geometrically symmetrical spots on its lateral surface with respect to both a transverse and a longitudinal symmetry plane that divide the porous element approximately into two mirror-identical halves, respectively, and type H corresponds to a porous element 5 having an activator solution 6 arranged at two geometrically symmetrical areas with respect to a C2 rotational symmetry axis, i.e., 180° rotation, and a diagonal symmetry plane passing diagonally through the porous element 5 that divides the porous element approximately into two mirror-identical halves.

According to an embodiment of the present invention, the device comprises two porous elements 5 arranged consecutively, i.e., one after the other.

In such an embodiment, preferably, the porous elements 5 have an activator solution 6 that corresponds with pattern type E, wherein the activator solution 6 is loaded on both, top and bottom surfaces of the porous element 5.

To determine the percentage of activator, present in a composition 3 comprising 1g of diethyl malonate (DEM), after dispensing the same, different porous elements 5 were loaded with an activator solution 6, comprising a tertiary amine, following pattern types A to H.

For pattern type A, 50µl of an activator solution 6 comprising 5mg of the tertiary amine dissolved in IPA (in a relation 1:10) were dispensed into a UHMWPE to homogeneously and fully load the porous element 5. The porous element 5 was then let to primary evaporate the IPA for 30min at room temperature and then placed in an oven at 70°C for 30 additional minutes.

For pattern types B to H, an activator solution 6 comprising 5mg of the tertiary amine was dispensed at the corresponding spots by equally distributing the amount of solution thereon. In order to dispense the solution accordingly, micro syringes, micropipettes and other dispensing tools were used. The amine was absorbed into the porous element 5.

To evaluate the performance of each pattern, composition 3 comprising 1g of diethyl malonate (DEM) was placed in nine different devices according to the invention, eight comprising a different porous element 5 corresponding to pattern types A to H, respectively, and one comprising two porous elements 5 corresponding to pattern E, arranged consecutively. For each of the nine devices two dispensations of 300mg each were carried out in two different vials, samples 1 and 2, respectively.

A solution of 7% w/w of undecane with DEM was used as the Internal Standard Solution.

10mg of sample 1 and 10mg of sample 2 along with 10mg of Internal Standard Solution each, were each placed in a GC vial comprising 980mL of DCM. Samples were then analyzed by GC-FID to determine the percentage of activator present in each sample, using a GC-FID Shimatzu gc-2030 column HP5 equipment.

Table I shows the percentage of activator present in each sample and the set time, i.e., the time needed to polymerize and dry each sample.

**Table I.**

| | Activator (%) | | Set time (s) | |
|---|---|---|---|---|
| Pattern type | Sample 1 | Sample 2 | Sample 1 | Sample 2 |
| A | 5 | 20 | 170 | 210 |
| B | 11 | 21 | 92 | 131 |
| C | 9 | 8 | 98 | 140 |
| D | 7 | 31 | 97 | 123 |
| E | 18 | 30 | 90 | 120 |
| 2xE | 35 | 43 | 80 | 90 |
| F | 37 | 29 | 67 | 105 |
| G | 47 | 36 | 68 | 100 |
| H | 39 | 45 | 73 | 85 |

Recovery of the activator from each resulting film by GC confirmed that the discontinuous geometrically symmetrical pattern of the porous elements 5 corresponding to pattern types E to H, in particular 2xE to H, have a higher percentage of activator and a better activator distribution throughout different dispensations.

It was also demonstrated that the addition of a solvent after dispensing the activator solution 6 corresponding to pattern types B to H, i.e., an activator solution only comprising the tertiary amine helps with the penetration and distribution of the activator.

The solvent is to be applied at the same spot wherein the activator solution 6 has been loaded. If an activator solution 6 comprising the solvent and the activator was applied to the porous element 5 it was found that the activator was too diluted and the activation of the monomer was negatively affected, while applying the solvent at a later stage, i.e., once the activator solution was absorbed, improved penetration and distribution of the activator within the porous element 5 without affecting activation of the monomer.

Table II shows the percentage of activator present in each sample and the set time for porous elements 5 corresponding to pattern types F, G and H which were additionally loaded with 2.5 µl of IPA at each spot, once all the activator solution 6 was absorbed.

**Table II.**

| | Activator (%) | | Set time (s) | |
|---|---|---|---|---|
| Pattern type | Sample 1 | Sample 2 | Sample 1 | Sample 2 |
| F - IPA | 43 | 39 | 57 | 94 |
| G - IPA | 51 | 46 | 55 | 60 |
| H - IPA | 45 | 49 | 65 | 70 |

The pattern type, not only affects the set time but also the physical and mechanical properties of the resulting film, as shown in Table III below for which elongation was determined according to ASTMF D882, tensile strength was determined according to ASTMF 2458-05 and adhesion was determined, for an average of 10 films, by recording the resulting film each 8 hours until the majority of the film was lifted or cracked.

**Table III.**

| Pattern Type | Cure time (s) 1st dispensation | Cure time (s) 2nd dispensation | Exotherm (°C) | Elongation (mm) | Tensile (N) | Adhesion |
|---|---|---|---|---|---|---|
| G | 70+4 | 88+3 | 45+1 | 300+25 | 70N | 4 days |
| H | 70+6 | 90+6 | 49+6 | ±53 | 65N | 4 days |
| E | 80+5 | 87+4 | 52+5 | +33 | 50N | 2-3 days |
| B | 75+10 | 90+11 | 51+10 | +114 | 30N | 16h |
| A | 120+16 | 150+29 | 50+8 | 177+18 | 25N | 8h |

Activation using G and H pattern types show a much better performance and reliability than prior art pattern types used to activate cyanoacrylates corresponding to pattern types A and B.

As shown in Table IV, the porous element 5 has a porosity which is inversely proportional to the viscosity of the monomer and/or polymeric composition 3 to ensure a minimum dwell time. Best results are obtained when the dwell time is in the range of about 7 to14 seconds of the latter in the porous element 5. This dwell time provokes the need for the user to release the pressure on the outer body 1, allowing air bubbles to come inside the device through the porous element 5 and allowing a vigorous mixing between the activator solution, the already activated part of the monomeric and/or polymeric composition and the non-activated part of the monomeric and/or polymeric composition, such that the composition contacts and mixes with the activator solution 6 sufficiently to initiate polymerization.

It was demonstrated that for monomeric and/or polymeric compositions 3 having high viscosity and porous elements 5 having small porous size, the dwell times of the monomeric and/or polymeric composition 3 within the porous element 5 were too long, leading to polymerization within the porous element 5 and causing blockage of the latter. While for monomeric and/or polymeric compositions 3 having low viscosity and porous elements 5 having high porous size, dwell times were too short, leading to poor activation.

**Table IV.**

| Composition Viscosity (cP) | Porosity (µm) |
|---|---|
| 10 | 10-30 |
| 40 | 30-70 |
| 100 | 70-120 |
| 150 | Cellulose acetate type |
| 200 | Polyurethane type |

The present invention also provides a method for activating electron-deficient olefin monomers, such as methylene malonate monomers. The method comprises providing a monomer formulation comprising at least one electron-deficient olefin monomer; providing at least one porous element having an activator solution therein; and forcing, at least once, the monomer formulation through the at least one porous element to contact the activator solution.

The activator solution 6 comprises at least one activator.

The activator solution 6 is arranged in the at least one porous element 5 in a predetermined discontinuous geometrically symmetrical pattern that ensures even and efficient activation of the monomeric and/or polymeric composition 3.

In an embodiment according to the method of the present invention, the activator solution 6 is arranged on the top and bottom surfaces of the porous element 5.

In an alternate embodiment, the activator solution 6 is arranged in the porous element 5 at two geometrically symmetrical spots on its lateral surface with respect to a transverse symmetry plane that divides the porous element approximately into two mirror-identical halves.

In a further embodiment, the activator solution 6 is arranged in the porous element 5 at four geometrically symmetrical spots on its lateral surface with respect to a transverse and a longitudinal symmetry plane that divide the porous element approximately into two mirror-identical halves, respectively.

In yet another embodiment, the activator solution 6 is arranged at two geometrically symmetrical areas with respect to a C2 rotational symmetry axis, i.e., 180° rotation, and a diagonal symmetry plane passing diagonally through the porous element 5 that divides the porous element approximately into two mirror-identical halves.

The porous element 5 has a porosity which is inversely proportional to the viscosity of the monomer and/or polymeric composition 3 to ensure a minimum residence time of the latter in the porous element 5, such that it contacts and mixes with the activator solution 6 sufficiently to initiate polymerization.

It has also been contemplated that the method of the present invention be carried out by means of a system for use in a laparoscopic intervention, such system comprising a pistol, a syringe loaded with the monomeric and/or polymeric composition 3 and a flexible tube comprising at an end thereof at least one porous element 5 loaded with an activator solution 6.

## Claims

1. A device for dispensing a tissue adhesive, said device comprising:
- an outer body (1) made of a flexible material;
- a monomeric and/or polymeric composition arranged inside said outer body (1);
- an applicator tip (4) attached to said outer body (1); and
- at least one porous element (5), arranged inside said applicator tip (4), comprising an activator solution arranged thereon and/or therein,
wherein the monomer and/or polymeric composition comprises at least one electron-deficient olefin-based monomer having at least one electron withdrawing group;
wherein the activator solution comprises a at least one activator;
**characterized in that** the activator solution is arranged on and/or in the at least one porous element (5) in a predetermined discontinuous geometrically symmetrical pattern that ensures an even and efficient activation of the monomeric and/or polymeric composition.

2. The device according to claim 1, wherein the activator solution is arranged on the top and bottom surfaces of the porous element (5) and the device comprises two porous elements 5 arranged consecutively one after the other.

3. The device according to claim 1, wherein the activator solution is arranged in the porous element (5) at two geometrically symmetrical spots on its lateral surface with respect to a transverse symmetry plane that divides the porous element approximately into two mirror-identical halves.

4. The device according to claim 1, wherein the activator solution is arranged in the porous element (5) at four geometrically symmetrical spots on its lateral surface with respect to a transverse and a longitudinal symmetry plane that divide the porous element approximately into two mirror-identical halves, respectively.

5. The device according to claim 1, wherein the activator solution is arranged at two geometrically symmetrical areas with respect to a C2 rotational symmetry axis and a diagonal symmetry plane passing diagonally through the porous element 5 that divides the porous element approximately into two mirror-identical halves.

6. The device according to any of the preceding claims, wherein the porous element (5) is arranged inside the applicator tip (4) in such a manner as to provide a tight fit between said porous element and said applicator tip.

7. The device according to any of the preceding claims, wherein the outer body (1) is made of aluminum.

8. The device according to any of claims 1 to 6, wherein said monomeric and/or polymeric composition is arranged inside said outer body (1) in a hermetically sealed leak-proof container (2).

9. The device according to claim 8, wherein the outer body (1) is made of a plastic material.

10. The device according to any of the preceding claims, wherein the porous element (5) has a porosity which is inversely proportional to the viscosity of the monomer and/or polymeric composition to ensure a dwell time of about 7 to 14 seconds of the composition in the porous element (5), such that it contacts and mixes with the activator solution sufficiently to initiate polymerization.

11. A method for activating electron-deficient olefin-based monomers, such as methylene malonate monomers, said method comprising the following steps:
a) providing a monomer and/or polymer composition comprising at least one electron-deficient olefin-based monomer having at least one electron withdrawing group;
b) providing at least one porous element (5) having an activator solution arranged thereon and/or therein; and
c) forcing, at least once, the monomeric and/or polymeric composition through the at least one porous element (5) to contact and mix with the activator solution in such a manner as to activate the monomeric and/or polymeric composition,
wherein the activator solution comprises at least one activator; and
wherein the activator solution is arranged on and/or in the porous element (5) in a predetermined discontinuous geometrically symmetrical pattern that ensures even and efficient activation of the monomeric and/or polymeric composition.

12. The method according to claim 11, wherein the activator solution is arranged on the top and bottom surfaces of the porous element (5).

13. The method according to claim 11, wherein the activator solution is arranged in the porous element (5) at two geometrically symmetrical spots on its lateral surface with respect to a transverse symmetry plane that divides the porous element approximately into two mirror-identical halves.

14. The method according to claim 11, wherein the activator solution is arranged in the porous element (5) at four geometrically symmetrical spots on its lateral surface with respect to a transverse and a longitudinal symmetry plane that divide the porous element approximately into two mirror-identical halves, respectively.

15. The method according to any of claims 11 to 14, wherein the porous element (5) has a porosity which is inversely proportional to the viscosity of the monomer and/or polymeric composition to ensure a dwell time of about 7 to14 seconds of the composition in the porous element (5), such that it contacts and mixes with the activator solution sufficiently to initiate polymerization.

## Patentansprüche

1. Vorrichtung zur Ausgabe eines Gewebeklebstoffs, wobei die Vorrichtung Folgendes umfasst:
- einen Außenkörper (1), der aus einem flexiblen Material hergestellt ist;
- eine monomere und/oder polymere Zusammensetzung, die innerhalb des Außenkörpers (1) angeordnet ist;
- eine Applikatorspitze (4), die an dem Außenkörper (1) befestigt ist; und
- mindestens ein poröses Element (5), das innerhalb der Applikatorspitze (4) angeordnet ist, das eine darauf und/oder darin angeordnete Aktivatorlösung umfasst,
wobei die Monomer- und/oder polymere Zusammensetzung mindestens ein elektronenarmes Monomer auf Olefinbasis mit mindestens einer elektronenziehenden Gruppe umfasst;
wobei die Aktivatorlösung mindestens einen Aktivator umfasst;
**dadurch gekennzeichnet, dass** die Aktivatorlösung auf und/oder in dem mindestens einen porösen Element (5) in einem vorbestimmten diskontinuierlichen geometrisch symmetrischen Muster angeordnet ist, das eine gleichmäßige und effiziente Aktivierung der monomeren und/oder polymeren Zusammensetzung gewährleistet.

2. Vorrichtung nach Anspruch 1, wobei die Aktivatorlösung auf der Ober- und Unterfläche des porösen Elements (5) angeordnet ist und die Vorrichtung zwei hintereinander angeordnete poröse Elemente (5) umfasst.

3. Vorrichtung nach Anspruch 1, wobei die Aktivatorlösung in dem porösen Element (5) an zwei geometrisch symmetrischen Stellen auf dessen Seitenfläche in Bezug auf eine Quersymmetrieebene angeordnet ist, die das poröse Element annähernd in zwei spiegelgleiche Hälften teilt.

4. Vorrichtung nach Anspruch 1, wobei die Aktivatorlösung in dem porösen Element (5) an vier geometrisch symmetrischen Stellen auf dessen Seitenfläche in Bezug auf eine Quer- und eine Längssymmetrieebene angeordnet ist, die das poröse Element jeweils annähernd in zwei spiegelgleiche Hälften teilen.

5. Vorrichtung nach Anspruch 1, wobei die Aktivatorlösung an zwei geometrisch symmetrischen Bereichen in Bezug auf eine C2-Rotationssymmetrieachse und eine diagonal durch das poröse Element (5) verlaufende Symmetrieebene angeordnet ist, die das poröse Element annähernd in zwei spiegelgleiche Hälften teilt.

6. Vorrichtung nach einem der vorhergehenden Ansprüche, wobei das poröse Element (5) innerhalb der Applikatorspitze (4) derart angeordnet ist, dass ein fester Sitz zwischen dem porösen Element und der Applikatorspitze bereitgestellt wird.

7. Vorrichtung nach einem der vorhergehenden Ansprüche, wobei der Außenkörper (1) aus Aluminium hergestellt ist.

8. Vorrichtung nach einem der Ansprüche 1 bis 6, wobei die monomere und/oder polymere Zusammensetzung innerhalb des Außenkörpers (1) in einem hermetisch abgedichteten auslaufsicheren Behälter (2) angeordnet ist.

9. Vorrichtung nach Anspruch 8, wobei der Außenkörper (1) aus einem Kunststoffmaterial hergestellt ist.

10. Vorrichtung nach einem der vorhergehenden Ansprüche, wobei das poröse Element (5) eine Porosität aufweist, die umgekehrt proportional zur Viskosität der Monomer- und/oder polymeren Zusammensetzung ist, um eine Verweilzeit der Zusammensetzung in dem porösen Element (5) von etwa 7 bis 14 Sekunden zu gewährleisten, so dass sie mit der Aktivatorlösung in Kontakt kommt und sich ausreichend mit ihr vermischt, um die Polymerisation einzuleiten.

11. Verfahren zur Aktivierung von elektronenarmen Monomeren auf Olefinbasis, wie Methylenmalonatmonomeren, wobei das Verfahren die folgenden Schritte umfasst:
a) Bereitstellen einer Monomer- und/oder Polymerzusammensetzung, die mindestens ein elektronenarmes Monomer auf Olefinbasis mit mindestens einer elektronenziehenden Gruppe umfasst;
b) Bereitstellen mindestens eines porösen Elements (5) mit einer darauf und/oder darin angeordneten Aktivatorlösung; und
c) mindestens einmaliges Zwängen der monomeren und/oder polymeren Zusammensetzung durch das mindestens eine poröse Element (5), um mit der Aktivatorlösung in Kontakt zu kommen und sich mit dieser zu vermischen, so dass die monomere und/oder polymere Zusammensetzung aktiviert wird,
wobei die Aktivatorlösung mindestens einen Aktivator umfasst; und
wobei die Aktivatorlösung auf und/oder in dem porösen Element (5) in einem vorbestimmten diskontinuierlichen geometrisch symmetrischen Muster angeordnet ist, das eine gleichmäßige und effiziente Aktivierung der monomeren und/oder polymeren Zusammensetzung gewährleistet.

12. Verfahren nach Anspruch 11, wobei die Aktivatorlösung auf der Ober- und Unterfläche des porösen Elements (5) angeordnet ist.

13. Verfahren nach Anspruch 11, wobei die Aktivatorlösung in dem porösen Element (5) an zwei geometrisch symmetrischen Stellen auf dessen Seitenfläche in Bezug auf eine Quersymmetrieebene angeordnet ist, die das poröse Element annähernd in zwei spiegelgleiche Hälften teilt.

14. Verfahren nach Anspruch 11, wobei die Aktivatorlösung in dem porösen Element (5) an vier geometrisch symmetrischen Stellen auf dessen Seitenfläche in Bezug auf eine Quer- und eine Längssymmetrieebene angeordnet ist, die das poröse Element jeweils annähernd in zwei spiegelgleiche Hälften teilen.

15. Verfahren nach einem der Ansprüche 11 bis 14, wobei das poröse Element (5) eine Porosität aufweist, die umgekehrt proportional zur Viskosität der Monomer- und/oder polymeren Zusammensetzung ist, um eine Verweilzeit der Zusammensetzung in dem porösen Element (5) von etwa 7 bis 14 Sekunden zu gewährleisten, so dass sie mit der Aktivatorlösung in Kontakt kommt und sich ausreichend mit ihr vermischt, um die Polymerisation einzuleiten.

## Revendications

1. Dispositif pour la distribution d'un adhésif tissulaire, ledit dispositif comprenant :
- un corps externe (1) constitué d'un matériau souple ;
- une composition monomérique et/ou polymérique agencée à l'intérieur dudit corps externe (1) ;
- un embout applicateur (4) fixé audit corps externe (1) ; et
- au moins un élément poreux (5), agencé à l'intérieur dudit embout applicateur (4), comprenant une solution d'activateur agencée sur celui-ci et/ou dans celui-ci,
dans lequel la composition monomère et/ou polymère comprend au moins un monomère à base d'oléfine déficiente en électrons ayant au moins un groupe attracteur d'électrons ;
la solution d'activateur comprend au moins un activateur ;
**caractérisé en ce que** la solution d'activateur est agencée sur et/ou dans l'au moins un élément poreux (5) selon un motif discontinu géométriquement symétrique prédéterminé qui assure une activation uniforme et efficace de la composition monomérique et/ou polymérique.

2. Dispositif selon la revendication 1, dans lequel la solution d'activateur est agencée sur les surfaces supérieure et inférieure de l'élément poreux (5) et le dispositif comprend deux éléments poreux (5) agencés consécutivement l'un après l'autre.

3. Dispositif selon la revendication 1, dans lequel la solution d'activateur est agencée dans l'élément poreux (5) en deux points géométriquement symétriques sur sa surface latérale par rapport à un plan de symétrie transversal qui divise l'élément poreux approximativement en deux moitiés identiques en miroir.

4. Dispositif selon la revendication 1, dans lequel la solution d'activateur est agencée dans l'élément poreux (5) en quatre points géométriquement symétriques sur sa surface latérale par rapport à un plan de symétrie transversal et longitudinal qui divise l'élément poreux approximativement en deux moitiés identiques en miroir, respectivement.

5. Dispositif selon la revendication 1, dans lequel la solution d'activateur est agencée en deux zones géométriquement symétriques par rapport à un axe de symétrie rotationnel C2 et à un plan de symétrie diagonal traversant en diagonale l'élément poreux (5) qui divise l'élément poreux approximativement en deux moitiés identiques en miroir.

6. Dispositif selon l'une quelconque des revendications précédentes, dans lequel l'élément poreux (5) est agencé à l'intérieur de l'embout applicateur (4) de manière à fournir un ajustement serré entre ledit élément poreux et ledit embout applicateur.

7. Dispositif selon l'une quelconque des revendications précédentes, dans lequel le corps externe (1) est constitué en aluminium.

8. Dispositif selon l'une quelconque des revendications 1 à 6, dans lequel ladite composition monomérique et/ou polymérique est agencée à l'intérieur dudit corps externe (1) dans un récipient étanche hermétiquement scellé (2).

9. Dispositif selon la revendication 8, dans lequel le corps externe (1) est constitué en matière plastique.

10. Dispositif selon l'une quelconque des revendications précédentes, dans lequel l'élément poreux (5) a une porosité qui est inversement proportionnelle à la viscosité de la composition monomère et/ou polymère pour assurer un temps de séjour d'environ 7 à 14 secondes de la composition dans l'élément poreux (5), de telle sorte qu'il entre en contact et se mélange suffisamment avec la solution d'activateur pour amorcer la polymérisation.

11. Procédé d'activation de monomères à base d'oléfines déficientes en électrons, tels que les monomères de malonate de méthylène, ledit procédé comprenant les étapes suivantes :
a) fournir une composition monomère et/ou polymère comprenant au moins un monomère à base d'oléfine déficiente en électrons ayant au moins un groupe attracteur d'électrons ;
b) fournir au moins un élément poreux (5) ayant une solution d'activateur agencée sur celui-ci et/ou dans celui-ci ; et
c) forcer, au moins une fois, la composition monomérique et/ou polymérique à travers l'au moins un élément poreux (5) pour qu'elle entre en contact et se mélange avec la solution d'activateur de manière à activer la composition monomérique et/ou polymérique,
dans lequel la solution d'activateur comprend au moins un activateur ; et
dans lequel la solution d'activateur est agencée sur et/ou dans l'élément poreux (5) selon un motif discontinu géométriquement symétrique prédéterminé qui assure une activation uniforme et efficace de la composition monomérique et/ou polymérique.

12. Procédé selon la revendication 11, dans lequel la solution d'activateur est agencée sur les surfaces supérieure et inférieure de l'élément poreux (5).

13. Procédé selon la revendication 11, dans lequel la solution d'activateur est agencée dans l'élément poreux (5) en deux points géométriquement symétriques sur sa surface latérale par rapport à un plan de symétrie transversal qui divise l'élément poreux approximativement en deux moitiés identiques en miroir.

14. Procédé selon la revendication 11, dans lequel la solution d'activateur est agencée dans l'élément poreux (5) en quatre points géométriquement symétriques sur sa surface latérale par rapport à un plan de symétrie transversal et longitudinal qui divise l'élément poreux approximativement en deux moitiés identiques en miroir, respectivement.

15. Procédé selon l'une quelconque des revendications 11 à 14, dans lequel l'élément poreux (5) a une porosité qui est inversement proportionnelle à la viscosité de la composition monomère et/ou polymère pour assurer un temps de séjour d'environ 7 à 14 secondes de la composition dans l'élément poreux (5), de telle sorte qu'il entre en contact et se mélange suffisamment avec la solution d'activateur pour amorcer la polymérisation.
